Europäisches Patentamt

European Patent Office

Office européen des brevets

(19)

(11) Numéro de publication: **0 160 585**

**A1**

(12)

# DEMANDE DE BREVET EUROPEEN

(21) Numéro de dépôt: 85400387.8

(22) Date de dépôt: 28.02.85

(51) Int. Cl.⁴: **A 61 F 5/14**
**A 43 B 7/28**

(30) Priorité: 01.03.84 FR 8403221

(43) Date de publication de la demande:
06.11.85 Bulletin 85/45

(84) Etats contractants désignés:
AT BE CH DE GB IT LI LU NL

(71) Demandeur: SOCIETE INDUSTRIELLE DE PRODUCTION
DE SYSTEME ENERGETIQUES Dite SIPSE
Zone artisanale
F-27600 Gaillon(FR)

(72) Inventeur: Denis, Marc
Zone artisanale
F-27600 Gaillon(FR)

(74) Mandataire: Martin, Jean-Jacques et al,
Cabinet REGIMBEAU 26, Avenue Kléber
F-75116 Paris(FR)

(54) Procédé de réalisation d'une semelle correctrice et/ou d'assistance par moulage.

(57) La présente invention concerne la réalisation d'une semelle correctrice et ou d'assistance par moulage.

Dans un bloc de pâte à modeler, on réalise une empreinte de plante de pied, que l'on corrige éventuellement avant d'y couler une forme rigide (23) ; après démoulage de cette dernière, on y marque en relief un contour (26) de semelle à réaliser puis on reporte ce contour sous forme d'un marquage en creux (27) de l'empreinte ; on découpe le bloc selon le marquage (27) pour en extraire une partie centrale ; après avoir reinséré la forme rigide (23) dans la partie de l'empreinte subsistant sur le bloc, on coule au moins une couche (32) sur la forme (23), à l'intérieur du bloc, pour former la semelle que l'on demoule ensuite.

On peut réaliser une semelle au cours d'une seule seance en présence d'un patient ; la forme rigide (23), conservée, permet de fournir d'autres semelles sans necessiter de nouvelles visites du patient.

FIG. 7

Croydon Printing Company Ltd

PROCEDE DE REALISATION D'UNE SEMELLE CORRECTRICE ET/OU
D'ASSISTANCE PAR MOULAGE ; PRODUIT INTERMEDIAIRE ET
SEMELLE OBTENUS PAR LA MISE EN OEUVRE DE CE PROCEDE.

La présente invention concerne la réalisation d'une semelle correctrice et/ou d'assistance pour pied douloureux ou non, par moulage.

On entend par semelle d'assistance une semelle épousant la forme naturelle d'un pied, et par semelle correctrice une semelle présentant une forme différente de la forme naturelle du pied, et déterminée par un médecin.

Selon un procédé connu, décrit par exemple par le préambule du certificat d'utilité en France N° 2 471 181, on réalise une semelle correctrice et/ou d'assistance par la succession des étapes consistant à :

a) réaliser une empreinte d'une plante de pied dans une face approximativement plane d'un bloc d'une pâte à modeler,

b) mouler une forme rigide dans cette empreinte,

c) démouler cette forme, et à galber ensuite sur cette forme une feuille d'un matériau tel que du cuir, apte à se déformer plastiquement pour conserver ensuite le galbe désiré, puis à rigidifier cette feuille, par exemple par apport de résine synthétique dont on provoque ensuite le durcissement ou par collage sur une plaque de liège creusée complémentairement, puis à rapporter éventuellement des bossages correctifs sur la feuille pour ensuite recouvrir le tout d'une feuille de peau.

Ce procédé connu nécessite de nombreuses opérations, et se révèle de ce fait long et coûteux ; il présente en outre l'inconvénient de nécessiter l'intervention d'une part d'un médecin orthopédiste, prescrivant notamment l'emplacement et la forme des éventuels bossages à rapporter, mais qui n'est généralement pas en mesure de fabriquer la semelle lui-même, et d'autre part d'un technicien prothésiste qui réalise cette semelle en atelier en fonction des prescriptions du médecin ; cette double intervention a pour résultat un allongement du délai entre la prescription de la semelle, la possibilité pour le patient de l'essayer, la réalisation des corrections éventuelles par rectification des bossages rapportés, et la livraison définitive de la semelle.

Pour remédier à ces inconvénients, le certificat d'utilité précité a proposé de confectionner une semelle orthopédique par moulage direct sur un pied d'un patient, en utilisant un matériau initialement susceptible de se déformer plastiquement et d'être mis en forme, et en outre susceptible de se rigidifier rapidement.

Cette méthode offre effectivement l'avantage de la rapidité, d'une part en raison du mode de confection de la semelle par moulage et d'autre part du fait que ce moulage peut intervenir directement dans le cabinet du médecin ; elle nécessite cependant une présence du patient non seulement lorsque l'on réalise pour la première fois une semelle à son intention, mais également chaque fois qu'il est nécessaire de renouveler cette semelle.

Le but de la présente invention est de remédier à ces inconvénients, en proposant un procédé de réalisation de semelle correctrice et/ou d'assistance par moulage susceptible d'être mis en oeuvre par le médecin orthopédiste lui-même, c'est-à-dire demandant peu  de temps et peu de matériel, en laissant à la disposition du médecin ou du prothésiste une forme rigide susceptible d'être réutilisée pour le renouvellement de la semelle en dehors de la présence du patient ; en outre, l'invention a pour but d'atteindre ces objectifs sans pour autant être coûteuse de mise en oeuvre.

A cet effet, la présente invention propose un procédé de réalisation d'une semelle correctrice et/ou d'assistance par moulage reprenant les étapes a), b), c) ci-dessus, l'étape c) s'accompagnant de la préservation de l'empreinte, et caractérisé en ce qu'il comporte en outre la succession des étapes consistant, ensuite, à :

d) marquer en relief, sur la forme, un contour de semelle à réaliser,

e) réinsérer la forme dans l'empreinte, pour réaliser un marquage, en creux, dudit contour dans celle-ci, ce marquage délimitant une partie centrale de l'empreinte et une partie périphérique de celle-ci,

f) dégager la forme de l'empreinte portant ledit marquage en creux en préservant ces derniers,

g) découper le bloc le long dudit marquage en creux, de part en part approximativement perpendiculairement à ladite face, pour dégager une partie centrale de bloc portant la partie centrale de l'empreinte et une partie périphérique de bloc, portant la partie périphérique de l'empreinte,

h) séparer la partie centrale de bloc de la partie périphérique de bloc en préservant cette dernière et la partie périphérique de l'empreinte,

i) poser la partie périphérique de bloc sur un support par ladite face en réinsérant la forme dans la partie périphérique de l'empreinte,

j) mouler une semelle à l'intérieur de la partie périphérique de bloc, sur la forme,

k) démouler la semelle.

Lorsqu'il s'agit de réaliser une semelle correctrice, on modifie l'empreinte en fonction de prescriptions orthopédiques entre l'étape a) et l'étape b).

On remarque que l'on obtient, lors de l'étape d), une forme rigide qui pourra être réutilisée par la suite pour réaliser une nouvelle empreinte, avec marquage en creux du contour, dans une face approximativement plane d'un bloc de pâte à modeler, pour pratiquer à nouveau les étapes f) et suivantes.

Ainsi, après réalisation d'une première semelle, la réalisation de semelles en tout point identiques à celle-ci pourra être effectuée en dehors de la présence du patient.

L'utilisation, pour la réalisation de la forme rigide et de la semelle par moulage, de résines

synthétiques à durcissement rapide bien connues dans le
domaine des matières plastiques permettra de réaliser
ces semelles rapidement, sans matériel compliqué,
même lors de la réalisation de la première semelle qui
pourra être effectuée directement dans le cabinet de
travail du médecin orthopédiste, en présence du patient
qui pourra au cours d'une même séance se prêter à
l'étape a) de réalisation de l'empreinte et essayer la
semelle obtenue, et le cas échéant émettre des observations permettant de remettre immédiatement en oeuvre
le procédé en apportant, entre l'étape a) et l'étape
d), des modifications de l'empreinte différentes de
celles qui auront éventuellement été apportées lors
de la mise en oeuvre précédente du procédé.

La mise en oeuvre du procédé selon l'invention se révèle économique non seulement du fait du
caractère lui-même économique des matériaux utilisés,
et d'autre part du fait de sa grande rapidité de mise
en oeuvre, et de sa possibilité de mise en oeuvre par
une seule personne en un même lieu.

D'autres caractéristiques et avantages de
l'invention ressortiront de la description ci-dessous,
relative à un mode de mise en oeuvre non limitatif,
ainsi que des dessins annexés qui font partie intégrante de cette description.

- Les figures 1 à 3 illustrent, en des vues
en coupe par un même plan vertical, les étapes a) et
b) de la mise en oeuvre du procédé.

- La figure 4 montre la forme rigide,
marquée en relief du contour de semelle à réaliser,
obtenue à l'étape d).

- Les figures 5 à 7 montrent, en des vues en coupe par le même plan qu'aux figures 1 à 3, les étapes e) et suivantes de la mise en oeuvre du procédé.

- La figure 8 montre, en une vue en coupe analogue, la semelle obtenue et un mode de finition de celle-ci.

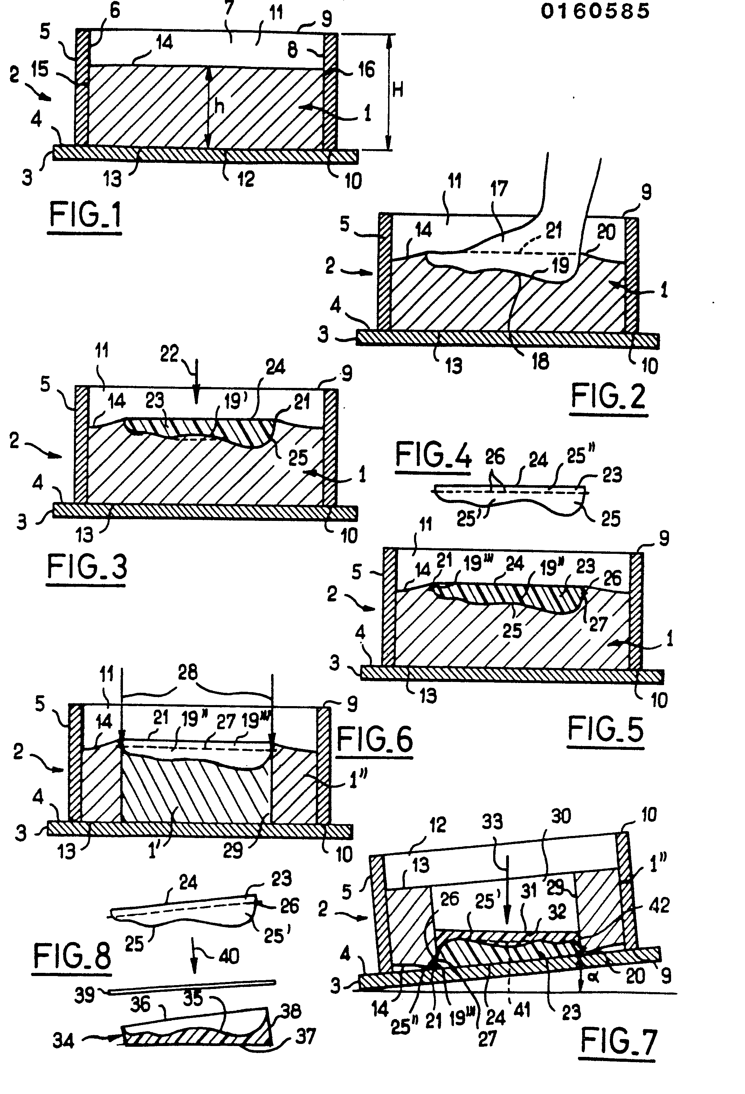

On se référera en premier lieu à la figure 1, où l'on a désigné par 1 un bloc d'une pâte à modeler ; ce bloc présente la forme générale d'un parallélépipède rectangle, et il est engagé de façon amovible dans une caisse rigide 2 définie d'une part par une plaque de base 3 présentant une face supérieure 4 et d'autre part par une ceinture 5 qui repose librement sur la face supérieure 4 de la plaque de base 3 par un bord inférieur plan 10 et présente intérieurement quatre faces planes dont seules trois sont visibles aux figures, où elles sont affectées respectivement des références 6, 7, 8; ces faces sont adjacentes deux à deux ainsi qu'à la face supérieure 4 de la plaque de base 3, et perpendiculaires deux à deux ainsi qu'à cette face 4 ; délimitées vers le bas par le bord inférieur 10 qui laisse la ceinture 5 intégralement ouverte entre elles (ouverture inférieure 12), elles sont par ailleurs délimitées vers le haut par un bord supérieur 9 de la ceinture 5, plan et parallèle au bord inférieur 10 de celle-ci, lequel bord 9 laisse également la ceinture 5 intégralement ouverte entre ces faces intérieures telles que 6 à 8 (ouverture supérieure 11).

Comme il ressort de la figure 1, préalablement à la mise en oeuvre du procédé, le bloc 1 présente une face inférieure plane 13 en contact, à plat, avec la face supérieure 4 de la plaque de base 3 à l'intérieur de la ceinture 5, au bord inférieur 10 de laquelle cette face 13 est de ce fait coplanaire, et une face supérieure 14 également plane et disposée en retrait vers l'intérieur de la ceinture 5 par rapport au bord supérieur 9 de celle-ci, la distance h séparant les faces 13 et 14 du bloc 1 étant inférieure à la distance H séparant les bords 9 et 10 de la ceinture 5 ; par ailleurs, le bloc 1 épouse les faces intérieures telles que 6 à 8 de la ceinture 5, sans adhérence de façon à pouvoir coulisser à l'intérieur de celle-ci, par quatre faces respectives telles que 15 (au contact de la face 6) et 16 (au contact de la face 8), planes et perpendiculaires deux à deux ainsi qu'aux faces 13 et 14.

Une première étape, la plus technique, de la mise en oeuvre du procédé consiste, en utilisant l'ensemble boîte 2 - bloc 1 tel qu'il vient d'être décrit, à introduire dans la ceinture 5, via l'ouverture supérieure 11 de celle-ci, le pied 17 auquel est destinée la semelle à réaliser, et à appliquer fermement ce pied, par sa plante 18, dans la face supérieure 14 du bloc 1 pour réaliser dans celle-ci une empreinte négative 19 de la plante 18 du pied ; le cas échéant, comme il est illustré à la figure 2, on supplée à une insuffisance de pénétration du pied 17 dans le bloc 1 en ramenant immédiatement autour du pied 17, sous forme d'un talus 20, de la pâte à modeler prélevée par exemple dans des zones de la face 14 immédiatement adjacentes à la ceinture 5 ; il convient néanmoins, lors de la pression du pied dans la pâte, de conserver la forme naturelle du pied et son orientation naturelle par rapport à la jambe ; à cet effet, notamment, on veille à éviter que les orteils soient relevés ou écartés et, la face supérieure 14 du bloc 1 étant supposée horizontale, on veille à conserver la verticalité de la jambe ; en outre, en appuyant plus précisément le côté extérieur du pied, dans toute la mesure du possible, on peut

corriger automatiquement, par la prise d'empreinte, des défauts de positionnement de la cheville ou, autrement dit, un affaissement ou un déport vers l'extérieur de la voûte plantaire ; de préférence, on donne au talus 20, autour du pied 17, une forme telle qu'il présente un bord supérieur 21 aussi plan que possible, et aussi parallèle que possible à la face 13 du bloc 1.

Ensuite, on extrait le pied 17 de l'empreinte 19 ainsi obtenue, laquelle est délimitée vers le haut et par rapport au talus 20, c'est-à-dire par rapport au reste de la face 14, par le bord 21.

Ensuite, soit sans modifier l'empreinte 19 s'il s'agit de réaliser une semelle d'assistance, soit après avoir modifié cette empreinte en fonction de prescriptions orthopédiques comme on l'a schématisé en 19' à la figure 3, on coule dans cette empreinte 19 une résine synthétique dite "matière plastique" à l'état liquide ou pâteux, comme on l'a schématisé en 22 à la figure 3, jusqu'à ce que l'empreinte 19 éventuellement modifiée comme il est indiqué en 19' soit remplie jusqu'au bord 21, conservé dans une position horizontale ; ensuite, selon la nature de la matière plastique ainsi introduite dans l'empreinte 19, soit on la laisse durcir dans cette empreinte, soit on la soumet à un traitement provoquant un tel durcissement dans l'empreinte, pour obtenir une forme positive 23 présentant une face supérieure 24 plane, définie par la limite de remplissage de l'empreinte 19, c'est-à-dire par coplanéarité avec le bord 21, et une face 25 tournée vers le bas et se raccordant de toute part à la face 24, laquelle face 25 reproduit exactement, en positif, l'empreinte 19 éventuellement corrigée comme il est indiqué en 19'.

A titre de matière plastique susceptible d'être utilisée pour réaliser la forme 23, on utilise de préférence une matière plastique à durcissement rapide ;
on peut également utiliser d'autres matières plastiques,
de même que l'on peut de façon plus générale utiliser
tout matériau aisément moulable et durcissable.

A titre d'exemple non limitatif, on a obtenu
de bons résultats en utilisant, pour réaliser une forme
23, une résine polyuréthane bi-composant, chargée :

- soit d'alumine hydratée, par exemple à
raison de 60 % en poids d'alumine pour 40 % en poids
de résine, dans le but de rendre la forme 23 obtenue
plus facile à travailler ultérieurement,

- soit de microsphères de verre creuses ou pleines, à raison
par exemple de 35 % en poids de microsphères pour 65 %
en poids de résine, dans ce même but et en outre pour
alléger cette forme.

En tout état de cause, le matériau destiné
à constituer la forme 23 est choisi tel que, après
durcissement, celle-ci soit parfaitement rigide.

Après durcissement du matériau constituant
la forme 23 dans l'empreinte 19, éventuellement modifiée
comme il est indiqué en 19', on démoule cette forme 23
en préservant l'empreinte puis, comme il est indiqué
à la figure 4, on marque en relief sur la forme, et
plus précisément sur la face 25 de celle-ci, un contour
26 de semelle à réaliser.

Ce marquage peut être effectué par différents
moyens, et par exemple par cloutage de la forme 23 ou
par pose d'agrafes dans celle-ci ; il peut être continu
ou discontinu, dès lors que le contour est parfaitement
déterminé en relief sur la forme.

Ensuite, on réinsère la forme 23, portant le marquage 26, dans l'empreinte 19, comportant toujours l'éventuelle modification indiquée en 19', pour reporter, sous forme d'un marquage en creux 27 dans le bloc 1, autour de l'empreinte, le contour 26 ainsi marqué en relief sur la face 25 de la forme 23 ; cette étape est illustrée à la figure 5 ; le marquage 27 délimite, dans l'empreinte 19 éventuellement modifiée comme il est indiqué en 19', une partie centrale 19" et une partie périphérique 19"'.

Ensuite, on dégage la forme 23, portant le contour en relief 26, de l'empreinte portant le marquage en creux 27 en préservant l'ensemble de ces éléments puis, comme il est indiqué en 28 à la figure 6, on découpe le bloc 1 le long du marquage en creux 27, de part en part approximativement perpendiculairement à la face 14, c'est-à-dire également à la face 13, pour dégager une partie centrale de bloc 1' portant la partie centrale 19" de l'empreinte et une partie périphérique de bloc 1" portant la partie périphérique 19"' de l'empreinte ; naturellement, par "empreinte", on entend ici cette empreinte telle qu'elle a éventuellement été modifiée comme indiqué en 19'.

On remarquera que pendant la totalité des étapes qui viennent d'être décrites, la boîte 2 et le bloc 1 restent dans la même position, les faces 4 et 14 ainsi que le bord 9 étant tournés vers le haut et approximativement horizontaux.

Au cours d'une étape suivante, on sépare la partie centrale de bloc 1' de la partie périphérique de bloc 1" en préservant au moins cette dernière, et notamment avec elle la partie périphérique 19"'de l'empreinte telle qu'elle a éventuellement été modifiée comme indiqué en 19'.

Ensuite, comme il est illustré à la figure 7, on réinsère la forme 23 dans la partie périphérique 19"' de l'empreinte 19, éventuellement modifiée comme il est indiqué en 19', en faisant coïncider le contour en relief 26 avec le contour 27 découpé, et l'on retourne l'ensemble formé par la ceinture 5, la partie périphérique de bloc 1' et la forme 23 ainsi placée, de façon à faire reposer l'ensemble sur la face supérieure 4 de la plaque de base 3 respectivement par le bord 9 de la ceinture 5, par la face 14 ou plus précisément par le bord 21 du talus 20, et par la face 24 de la forme 23 ; compte tenu de la différence entre les distances h et H, ce mouvement s'accompagne d'un coulissement de la partie périphérique 1" du bloc à l'intérieur de la ceinture 5.

Comme il ressort de la figure 7, la partie périphérique 1" du bloc, par la découpe 29 réalisée par l'opération 28, délimite alors une cavité 30 ouverte vers le haut par l'ouverture 12, alors tournée vers le haut, de la ceinture 5, et cette cavité 30 est fermée vers le bas par la forme 23, et plus précisément par une partie centrale 25' de cette face 25, délimitée périphériquement par le contour en relief 26 qui coïncide avec le contour 27, constituant la transition entre la découpe 29, que la forme 23 laisse dégagée, et la partie périphérique 19"' de l'empreinte 19 éventuellement modifiée comme il a été indiqué en 19', que la forme 23 épouse par contre étroitement par la partie 25" de sa face 25 située entre le contour 26 et sa face 24 (voir également la figure 4).

Ainsi, par l'ouverture 12, alors tournée vers le haut, de la ceinture 5, on peut introduire dans la cavité 30 tout matériau voulu et, dans un premier temps, on enduit d'un matériau d'étanchéification la jonction entre la découpe 29 et la partie centrale 25' de la face 25, c'est-à-dire des zones de l'une et l'autre jouxtant le marquage 27 et le contour 26 en coïncidence ; avantageusement, on utilise à cet effet un matériau dont on revêt l'intégralité de la partie centrale 25' de la face 25 et de la forme 23 et les zones adjacentes de la découpe 29 dans le but de faciliter le démoulage ultérieur de la semelle qui sera ensuite réalisée par moulage dans la cavité 30, comme il apparaîtra plus loin ; à titre d'exemple non limitatif, ce matériau d'étanchéité et de démoulage peut être constitué par de la vaseline Codex, d'autres matériaux pouvant naturellement être utilisés sans que l'on sorte pour autant du cadre de la présente invention.

Ensuite, on procède au moulage de la semelle dans la cavité 30 en coulant dans cette dernière, à l'état liquide ou pâteux, un matériau durcissable choisi notamment en fonction de la plus ou moins grande dureté ou de la plus ou moins grande molesse que l'on désire donner à la semelle.

A titre d'exemples non limitatifs, on peut utiliser un élastomère de polyuréthane éventuellement chargé de poudrette de caoutchouc naturel, un élastomère de silicone ou un élastomère de caoutchouc, un homme du métier pouvant aisément donner à ces produits la formulation appropriée selon la consistance à donner à la semelle ; naturellement, d'autres matériaux peuvent être utilisés sans que l'on sorte pour autant du cadre de la présente invention.

Préalablement à cette coulée, on peut cloisonner la cavité 30 afin de délimiter sur la partie centrale 25' de la face 25 de la forme 23 des zones prédéterminées, dans lesquelles on coule un matériau différent de celui que l'on coule dans les autres zones, afin de moduler les caractéristiques de la semelle réalisée ; on peut également couler successivement plusieurs couches de matériaux différents, étant bien entendu que le matériau coulé en premier remplira en priorité les zones concaves de la partie centrale 25' de la face 25 de la forme 23, comme par exemple la partie de celle-ci correspondant à la voûte plantaire, ce qui permettra, par le choix d'un matériau destiné à devenir comparativement plus dur comme matériau introduit en premier dans la cavité 30 puis d'un matériau destiné à devenir comparativement plus mou comme second matériau introduit dans cette cavité, de raidir comparativement la partie de la semelle correspondant à la voûte plantaire.

Les opérations de coulée et de durcissement dans la cavité 30 sont avantageusement effectuées en inclinant l'ensemble boîte 2 (c'est-à-dire plaque de base 3 et ceinture 5 retournée sur celle-ci) - partie périphérique 1" du bloc - forme 23, et plus précisément les faces 4 et 24 et le bord 21 coplanaires, d'un angle prédéterminé α, par rapport à l'horizontale, de façon à placer de façon prédéterminée, par rapport à la partie centrale 25' de la face 25 de la forme 23, la face supérieure horizontale 31 du matériau 32 coulé dans la cavité 30, ou les faces supérieures horizontales respectives des différents matériaux coulés successivement dans la cavité 30, lors de cette opération ; l'angle α peut varier d'une couche à l'autre lorsque plusieurs couches doivent être formées ; il est déterminé en fonction des prescriptions du médecin ; dans le cas d'une couche unique, ou dans le cas de la couche coulée en dernier lorsque plusieurs couches sont superposées, cet angle α est tel

qu'un plan défini par les images respectives, sur la face 25 de la forme 23, des quatre points d'appui fondamentaux du pied dans le cas d'un pied normal à cet égard, ou occupant par rapport à ces images une position déterminée par le médecin lorsqu'il n'en est pas ainsi, soit horizontal si bien que l'on peut déterminer la fin de la coulée par la constatation de la couverture de l'ensemble de ces images par le matériau constituant cette couche unique ou cette dernière couche, ce qui permet de donner à la semelle une épaisseur moyenne minimale, c'est-à-dire également une légèreté maximale.

En outre, en fonction des prescriptions, on peut noyer dans la couche unique ou dans l'une au moins des couches multiples, lors de la coulée, au moins un élément préfabriqué tel qu'une plaque raidisseuse de forme déterminée 41 ou autre.

Une fois la couche 32 unique coulée comme indiqué en 33 à la figure 7, ou lorsque l'une des couches multiples est coulée, on provoque son durcissement ou on la laisse se durcir librement en fonction du matériau utilisé ; si plusieurs couches doivent être coulées successivement, on procède ainsi après chaque coulée d'une couche.

On obtient ainsi, à l'intérieur de la cavité 10, la semelle désirée 34 que l'on démoule alors, c'est-à-dire que l'on dégage de la partie périphérique 1" du bloc et de la forme 23 ; on sépare de façon générale l'ensemble des éléments apparaissant sur la figure 7, en préservant la forme 23 revêtue du contour en relief 26 en vue d'une utilisation ultérieure, par un procédé consistant à :

- réaliser, de la façon décrite en référence à la figure 2, une empreinte de cette forme 23, avec marquage en creux du contour 26, dans une face approximativement plane telle que 14 d'un bloc de pâte à modeler tel que 1, puis découper ce dernier en une partie centrale et une partie périphérique, approximativement perpendiculairement à ladite face, comme il a été décrit en référence à la figure 6, puis séparer la partie centrale du bloc de la partie périphérique de bloc en préservant cette dernière, ainsi que la partie périphérique de l'empreinte qu'elle porte, puis poser la partie périphérique de bloc sur un support par ladite face en réinsérant la forme dans la partie périphérique de l'empreinte, puis à mouler une semelle à l'intérieur de la partie périphérique de bloc, sur la forme, en adoptant l'une ou l'autre des variantes décrites en référence à la figure 7, puis à démouler la semelle obtenue.

Ainsi, en archivant la forme 23 revêtue du marquage en relief 26, l'orthopédiste peut aisément fournir de nouvelles semelles à un patient, sans que ce dernier ait à nouveau à fournir une empreinte de son pied et sans que l'on ait à répéter l'éventuelle correction de cette empreinte.

Quant à lui, le bloc de pâte à modeler 1 peut être reconstitué à partir de la partie périphérique 1" et de la partie centrale 1', par exemple par malaxage, pour retrouver la forme d'un bloc parallélépipédique.

Après son démoulage, la semelle obtenue 34, présentant une face en creux 35 reproduisant en négatif la partie centrale 25' de la face 25 de la forme 23, délimitée par un bord plan 36 constituant l'image du contour 27 ou du marquage 26, une face de base plane 37 correspondant à la face supérieure 31 de la couche 32 ou des couches telles que 32, et une face périphérique 38 joignant la face 37 au bord 36 et formée par la zone 42 de contact de la couche 32 ou des couches telles que 32 avec la découpe 29 de la partie périphérique 1" du bloc, peut subir diverses opérations de finition.

Une finition de la face 35 peut consister en son revêtement au moyen d'une feuille de peau 39, la partie centrale 25' de la face 25 de la forme 23 étant avantageusement utilisée à cet effet comme poinçon de formage et de collage contre la face 35 de la semelle 34, comme on l'a schématisé en 40 à la figure 8 ; en ce qui concerne les faces 37 et 38, les travaux de finition peuvent consister par exemple en un taillage, en vue d'une adaptation au contour intérieur d'une chaussure.

Une semelle 34 obtenue par la mise en oeuvre du procédé qui vient d'être décrit, et la forme 25, revêtue du contour en relief 26 d'une telle semelle à réaliser constituent, comme le procédé qui vient d'être décrit, des caractéristiques de la présente invention.

On notera que cette dernière pourra connaître de nombreuses variantes ne sortant pas de son esprit ; en particulier, dès lors que la consistance de la pâte à modeler s'y prêtera, ou pourra se dispenser de la ceinture 5 de la boîte 2 décrite et représentée, dont seule sera conservée la plaque de base 3.

On remarquera enfin d'une part la grande précision et d'autre part la grande simplicité et la grande économie de mise en oeuvre du procédé selon l'invention, dûes notamment à l'utilisation du bloc de pâte à modeler portant l'empreinte à la fois comme moule permettant la réalisation d'une forme positive rigide archivable en vue d'une réutilisation, et comme moule permettant la réalisation d'un négatif souple, à savoir , la semelle.

REVENDICATIONS

1. Procédé de réalisation d'une semelle
correctrice et/ou d'assistance par moulage, comportant
la succession des étapes consistant à :

a) réaliser une empreinte (19) d'une plante
de pied (18) dans une face approximativement plane (14)
d'un bloc d'une pâte à modeler (1),

b) mouler une forme rigide (23) dans cette
empreinte (19),

c) démouler cette forme (23) en préservant
l'empreinte (19), caractérisé en ce qu'il comporte en
outre la succession des étapes consistant, ensuite, à :

d) marquer en relief sur la forme (23), un contour (26)
de semelle à réaliser,

e) réinsérer la forme (23) dans l'empreinte
(19),pour réaliser un marquage (27), en creux, dudit
contour (26) dans celle-ci,ce marquage   (27) délimitant
une partie centrale (19") de l'empreinte (19) et une
partie périphérique (19"') de celle-ci,

f) dégager la forme (23) de l'empreinte (19)
portant ledit marquage en creux (27) en préservant ces
derniers,

g) découper (28) le bloc (1) le long dudit
marquage en creux (27), de part en part approximativement
perpendiculairement à ladite face (14), pour dégager
une partie centrale de bloc (1'), portant la partie
centrale (19") de l'empreinte (19),et une partie périphérique de bloc (1"'), portant le partie périphérique
(19"') de l'empreinte (19),

h) séparer la partie centrale de bloc (1')
de la partie périphérique de bloc (1") en préservant
cette dernière  et la partie périphérique (19"') de
l'empreinte (19),

i) poser la partie périphérique de bloc (1") sur un support (3) par ladite face (14) en réinsérant la forme (23) dans la partie périphérique (19"') de l'empreinte (19),

j) mouler une semelle (34) à l'intérieur de la partie périphérique de bloc (1"), sur la forme (23

k) démouler la semelle (34).

2. Procédé selon la revendication 1, caractérisé en ce que, à l'étape k, on préserve la forme (23) portant en relief ledit contour (26), et on l'extrait de la partie périphérique de bloc (1") en vue d'une réutilisation ultérieure par un procédé consistant à réaliser une empreinte de la forme, avec marquage en creux du contour, dans une face approximativement plane d'un bloc de pâte à modeler, puis pratiquer à nouveau les étapes f, g, h, i, j, k.

3. Procédé selon l'une quelconque des revendications 1 et 2, caractérisé en ce que, entre l'étape a et l'étape b, on modifie (19') l'empreinte (19) en fonction de prescriptions orthopédiques.

4. Procédé selon l'une quelconque des revendications 1 à 3, caractérisé en ce que, à l'étape b, on moule la forme rigide (23) par coulée (22) d'une matière durcissable à l'état liquide ou pâteux dans l'empreinte (19), puis durcissement de cette matière dans l'empreinte (19).

5. Procédé selon l'une quelconque des revendications 1 à 4, caractérisé en ce que, à l'étape j, on moule la semelle (34) par coulée (33) d'au moins une couche de matière durcissable (32) à l'état liquide ou pâteux à l'intérieur de la partie périphérique (1") de bloc, sur la forme (23), puis durcissement de cette matière (32) à l'intérieur de cette partie périphérique de bloc (1"), sur cette forme (23).

6. Procédé selon la revendication 5, caractérisé en ce que, à l'étape j, on cloisonne des zones de la forme (23), à l'intérieur de la partie périphérique (1") de bloc, préalablement à ladite coulée et l'on effectue cette dernière en coulant des couches de matières durcissables différentes dans lesdites zones.

7. Procédé selon l'une quelconque des revendications 5 et 6, caractérisé en ce que, entre l'étape i et l'étape j, on réalise une étanchéité entre la forme (23) et la partie périphérique (1") de bloc.

8. Procédé selon l'une quelconque des revendications 5 à 7, caractérisé en ce que l'on pratique l'étape j en donnant au support (3), au moins une orientation déterminée (α) en fonction de prescriptions orthopédiques.

9. Procédé selon l'une quelconque des revendications 5 à 8, caractérisé en ce que l'on incorpore au moins un élément préfabriqué (4) à ladite couche (32) ou auxdites couches lors de la coulée (33).

10. Procédé selon l'une quelconque des revendications 1 à 9, caractérisé en ce que, entre l'étape i et l'étape j, on revêt la forme (23) et la partie périphérique (1") de bloc d'un matériau de démoulage.

11. Procédé selon l'une quelconque des revendications 1 à 10, caractérisé en ce que, à l'étape k, on préserve la forme (23) portant en relief ledit contour (26) et l'on utilise cette forme (23) à titre de poinçon pour former sur la semelle (34) une feuille d'un matériau de revêtement (39).

12. Forme rigide portant, marqué en relief, un contour de semelle à réaliser, telle qu'elle est obtenue à l'étape d du procédé selon l'une quelconque des revendications 1 à 11.

13. Semelle correctrice et/ou d'assistance obtenue par la mise en oeuvre du procédé selon l'une quelconque des revendications 1 à 11.

0160585

FIG.1

FIG.2

FIG.3

FIG.4

FIG.5

FIG.6

FIG.7

FIG.8

## DOCUMENTS CONSIDERES COMME PERTINENTS

| Catégorie | Citation du document avec indication. en cas de besoin. des parties pertinentes | Revendication concernee | CLASSEMENT DE LA DEMANDE (Int Cl 4) |
|---|---|---|---|
| A | US-A-2 589 241  (L.B. GALHOUSE et al.) | 1-5,10 ,12,13 | A 61 F    5/14<br>A 43 B    7/28 |
| A | DE-C-  800 366  (A. PROFFEN) | 1,3,4, 11-13 | |
| A | DE-A-2 446 483  (H. WECK) | 1,3,4, 11 | |
| A | US-A-2 565 758  (S. COVINO) | 1,10- 13 | |
| A | US-A-2 120 987  (A.E. MURRAY) | 1,4,6, 8,12, 13 | |
| A | FR-A-2 304 299  (SANIPED) | 1,9 | **DOMAINES TECHNIQUES RECHERCHES (Int Cl 4)**<br><br>A 61 F<br>A 43 B |
| A | US-A-1 914 049  (J.H. SMITH) | 1,3,9 | |
| A | FR-A-  789 393  (E.E. KOST) | 1,10, 11 | |
| A | US-A-3 825 017  (J.E. SCRIMA) | 1,12, 13 | |

Le present rapport de recherche a ete etabli pour toutes les revendications

| Lieu de la recherche | Date d achevement de la recherche | Examinateur |
|---|---|---|
| LA HAYE | 22-05-1985 | WOLF C.H.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur. mais publié à la date de dépôt ou après cette date
D : cité dans la demande
L : cité pour d autres raisons

& : membre de la même famille. document correspondant

OEB Form 1503 03 82